# EUROPEAN PATENT SPECIFICATION

(11) **EP 1 351 673 B1**
(45) Date of publication and mention of the grant of the patent: **22.08.2007**
(21) Application number: 01272465.4
(22) Date of filing: 30.11.2001
(51) Int. Cl.: A61K 31/195, A61K 47/42, A61P 9/04

(54) **COMPOSITIONS OF STABLE T3 AND USE THEREOF**
STABILE T3 ZUSAMMENSETZUNGEN UND IHRE ANWENDUNG
COMPOSITIONS DE T3 STABLE ET LEURE UTILISATION

(30) Priority: 21.12.2000 US 257666 P; 12.03.2001 US 327569 P
(43) Date of publication of application: 15.10.2003
(73) Proprietor: Resuscitation Technologies, LLC, Santa Monica, CA 90403 (US)
(72) Inventor: KLEIN, Irwin, Dept. of Endocrinology, Manhasset, NY 11030 (US); OJAMAA, Kaie, Dept. of Endocrinology, Manhasset, NY 11030 (US); RUBIN, Leo, Suffern, NY 10901 (US)
(74) Representative: Wright, Simon Mark
(86) International application number: PCT/US2001/044855
(87) International publication number: WO 2002/051403

(56) References cited:
- EP-A- 0 326 618
- EP-A- 0 337 467
- EP-A- 0 583 717
- GB-A- 2 240 474
- US-A- 5 795 789

## Description

The present invention relates generally to a stable aqueous formulation of T₃ which can be used for treating patients to restore effective cardiac function.

### BACKGROUND OF THE INVENTION

Serum albumin is a serum protein fraction involved in maintaining blood osmotic pressure and is used as a plasma substitute in shock treatment. Serum Albumin also contributes to many body transport and regulatory processes.

Like many other substances, such as N-oxy trimethyl amines, amino acids, alkylated amino acids, and sugars, serum albumin is used as a protein protectant to stabilize proteins against denaturation and to preserve enzymatic activity as well as in formulation of biomedicals. U.S. Patent No. 5,876,992 discloses the use of serum albumin, together with disaccharides or their derivatives to stabilize proteins. Serum albumin has been used to preserve the integrity of urinary proteins. U.S. Patent No. 5,679,318. In addition, serum albumin solubilizes paclitaxel in aqueous solution WO 00/06152.

Thyroid hormones include the L-forms of thyroxine (4-(4-Hydroxy-3,5-diiodophenyl)-3,5-diidotyrosine; hereinafter T₄) and 3, 5, 3'-triiodothyronine (T₃). They can be obtained from natural sources, such as bovine thyroid glands or synthesized. U.S. Patent No. 2,803,654.

Thyroid hormones administered to patients with cardiovascular compromise restore or improve cardiac rhythm and function. Thyroid hormones increase heart rate and heart beat force thus increasing cardiac output and are found to be significantly decreased during cardiac arrest. Wortsman et al. (1987) Arch. Intern. Med. 147:245-248. An infusion of thyroid hormone effects cardiac resuscitation in patients undergoing cardiac arrest, cardiac standstill, electro-mechanical dissociation and a variety of other cardiac conditions (GB2 240 474). The effect of thyroid hormone is almost immediate and occurs even where standard treatments have failed. Thyroid hormones are also therapeutically effective in other cardiac indications such as cardiomyopathies and bradyarrhythmias.

Of the thyroid hormones, T₃ is normally synthesized in smaller quantities than T₄ and presents in blood and the thyroid gland. However, on a molecular basis, T₃ is more potent and the onset of its effect is more rapid than T₄ and is synthesized in the thyroid gland and by metabolism of T₄ in peripheral tissues by the enzyme 5' deiodinase. T₄ has been the preferred thyroid hormone in clinical use today, largely due to its availability and relatively long half-life of 6-7 days because T₄ binds avidly to thyroxine-binding globulin in human serum and is thus protected from metabolism and excretion. T₃ has higher potency and more rapid effect than T₄ in resuscitate patients undergoing cardiac arrest. However, T₃ is unstable in aqueous solution with an extremely short half-life. This short half-life has limited the application of T₃ in treating patients, especially in emergency situations where the injection of an aqueous thyroid hormone solution is required.

Although a stable T₃ formulation is desirable and needed in treating patients with heart disease, there has been no report or actual use of a stable aqueous T₃ formulation. The present invention addresses this longstanding need and desire in the art.

### SUMMARY OF THE INVENTION

The invention provides a composition comprising between 0.01 mg/ml and 1.0 mg/ml T₃, serum albumin and water wherein the stability of T₃ is increased.

The invention further provides use of a composition according to the invention for the manufacture of a medicament for treating a patient with cardiac arrest, or with cardiac electrical standstill, to restore effective cardiac function.

The invention encompasses a stable aqueous pharmaceutical composition containing T₃, serum albumin and water. Dried samples for reconstitution are also encompassed by the invention as are various pharmaceutical preparations.

The invention can be used in a method for emergency treatment of a patient with cardiac arrest, and with cardiac electrical standstill, to restore effective cardiac function, by administering to the patient a therapeutically effective amount of a pharmaceutical composition of T₃, serum albumin and water.

### BRIEF DESCRIPTION OF THE DRAWINGS

Figure 1 describes mortality rate as a function of the level of thyroid hormone during acute myocardial infarction.
Figure 2 shows hemodynamic data after defibrillation (5 min-VF).
Figure 3 describes detailed procedures for testing the effectiveness of T₃, (Protocol 1).
Figure 4(A-H) shows the effects of T₃ₐ injections on blood T₃ levels.
Figure 5 shows left ventricle (LV) pressure after T₃ₐ injection.
Figure 6 provides results of several animal model studies of T₃ₐ. In Figures 6G and H, * indicates where ECG data points do not totally correlate with the pressures below; it is only shown to distinguish between ventricular fibrillation (VF) and sinus rhythm here.
Figure 7(A-I) shows the serum total T₃ levels (ng/dl) as a function of time after T₃ₐ injection during cardiac resuscitation in dogs.
Figure 8 is a graph depicting pH-dependent T₃ and T₃ₐ stability measured over 13 months.
Figure 9 is a graph depicting serum T₃ levels after T₃ injection during cardiac resuscitation in dogs.
Figure 10 is a graph depicting serum T₃ levels after a single 5 µg/kg dose of T₃.
Figure 11 is a graph depicting serum T₃ after 100 µg/kg bolus dose of T₃
Figure 12 is a graph depicting the half-life of serum T₃ after a single injection.
Figure 13 is a graph depicting ¹²⁵I-T₃ uptake into rat neonatal myocyte-nuclear fraction.

### DETAILED DESCRIPTION OF THE INVENTION

The invention provides a stable liquid composition containing T₃ or an analog thereof, serum albumin and water, where the T₃ has a half-life of at least five days at a temperature range of-30°C and 70°C, preferably -10°C and 50°C and more preferably 0°C and 30°C. The compositions described herein are suitable for use in any condition for which T₃ is indicated. The invention can be used for treatment, of a patient with cardiac arrest and cardiac electrical standstill to restore effective cardiac function, comprising administering to the patient a therapeutically effective amount of the composition.

In one embodiment of the invention, the T₃ in the composition has a half-life of or at least two weeks, preferably at least one month, more preferably at least three months, even more preferably at least six months and most preferably at least one year.

In another embodiment of the invention, the composition has a T₃ and serum albumin ratio of between 0.001 and 0.1, preferably between 0.002 and 0.05. In another embodiment of the invention, the serum albumin is a human serum albumin or a bovine serum albumin. In yet another embodiment of the invention, T₃ in the composition has a concentration of between 0.01 mg/ml and 1.0 mg/ml, preferably between 0.02 mg/ml and 0.8 mg/ml and more preferably between 0.1 mg/ml and 0.5 mg/ml.

The compositions can further comprise other pharmaceutically effective or acceptable compositions such as epinephrine, adrenaline and any excipients. Pharmaceutically acceptable excipients are isotonic and include, without limitation, saline and phosphate buffered saline.

The increased stability and solubility under physiologic conditions of T₃ in the composition of the present invention allows the use and manufacture of a variety of compositions not previously available. Methods of making such compositions are known in the art, but have been previously unavailable for use with T₃. Various compositions for different delivery means are provided herein. These compositions include, without limitation, those suitable for use in intravenous, direct cardiac, parenteral, mucosal, intranasal, by-inhalation and buccal administration.

The compositions are particularly suitable for use in by-inhalation delivery. Aqueous solutions are far more effective at drug delivery than dry formulations but are not often used due to the instability and tendency of many drugs to aggregate in solution. Methods of making compositions for by-inhalation etc are known in the art. U.S. Patent No. 5,011,678 describes suitable compositions containing a pharmaceutically active substance, a biocompatible amphiphilic steroid and a biocompatible carbon propellant. U.S. Patent No. 5,006,343 describes suitable compositions containing liposomes, pharmaceutically active substances and an amount of alveolar surfactant protein to enhance transport of the liposomes across a pulmonary surface.

The composition may be administered by direct injection to a heart cavity of the patient, or direct parenteral injection into a central venous line of the patient, or is administered by parenteral injection or parenteral intravenous injection, or directly to the pulmonary system of the patient, or directly to the pulmonary system by direct endotracheal injection, or directly to the pulmonary system by infusion through a respiratory airway of the patient, or in at least one rapid bolus injection. The composition can also be administered via drip from an intravenous line.

As used herein, the term "therapeutically effective amount" means a dosage of T₃ preferably between 0.1 and 20 µg/kg of body weight, preferably between 0.2 and 10 µg/kg and more preferably between 0.3 and 5 µg/kg although dosages of up to at least 100 µg/kg are effective. Surprisingly, dosages of 5 µg/kg and 100 µg/kg appear to result in similar physiologic distribution. Therefore, the composition allows for use of a low concentration of T₃ to achieve a therapeutically effective endpoint. A therapeutically effective amount is also used to describe the amount required to treat any condition that responds to T₃. Preferably the amount is sufficient restore effective cardiac function in a patient in need thereof.

Throughout the present application, T₃ₐ represents a composition of T₃ and serum albumin. T₃ₐ has a pH range of 2.5 to 11.5, preferably 4.0 to 10 more preferably 6.0 to 8.0 and most preferably 6.5 to 7.5.

The invention also includes packaged combinations which may be used for parenteral administration of liquid T₃ formulation to patients with cardiovascular compromise. Such packaged combinations include a device suitable for injecting T₃ formulation alone or in combination either dissolved in a physiologically acceptable diluent in combination with a physiologically acceptable diluent for dilution just prior to use. The diluent can be formulated to additionally contain various therapeutically effective substances which enhance the heart functions including but not limited to calcium and magnesium in therapeutically acceptable amounts.

It is apparent from the following examples that T₃ₐ effects cardiac resuscitation when other standard treatments have failed. T₃ₐ did not cause any symptoms of hyperthyroidism in the treated dogs. T₃ is the preferred thyroid hormone for use in humans despite the fact that T₃ has heretofore not been used clinically, as T₃ has high specific activity and does not persist after administration so as to decrease or eliminate the need for subsequent β-blocker therapy.

The following examples are provided to illustrate but not limit the invention. The examples show the stability of T₃ₐ compared to T₃ alone, particularly in a pH neutral environment and the cardiac benefit of T₃ in the immediate period following drug administration during cardiopulmonary resuscitation.

### Example 1

### Thyroid hormones during acute myocardial infarction as an indicator for mortality

In severe illness, including cardiac disease, the thyroid hormone system may be temporarily downgraded. This "sick euthyroid syndrome" has been regarded as an adaptive response to conserve energy. However, thyroid hormone also reduces systemic vascular resistance, improves systolic and diastolic function and has beneficial effects on platelet function and lipids. Recent experimental data indicate thyroid hormone treatment is of value for some patients with cardiac disease.

Thyroid hormone values during acute myocardial infarction are of importance for the prognosis. A comparison of thyroid hormone levels on arrival to the CCU in 331 consecutive pts (age 68±12 yrs) with acute myocardial infarction to a healthy control shows a significant down-regulation of the thyroid hormone system. In a multivariate analysis considering age, sex, thyroid hormones, CKB, previous myocardial infarction, angina, heart failure and diabetes, a serum concentration of reverse T₃ (rT₃) over the median value 0.41 nmol/L was identified as an independent risk factor after myocardial infarction. The odds ratio for death within the first month was 10,8 (95% conf. interval 2,3-51,7 p 0,003) and within one year 3,0 (1,2-7,3 p 0,02).

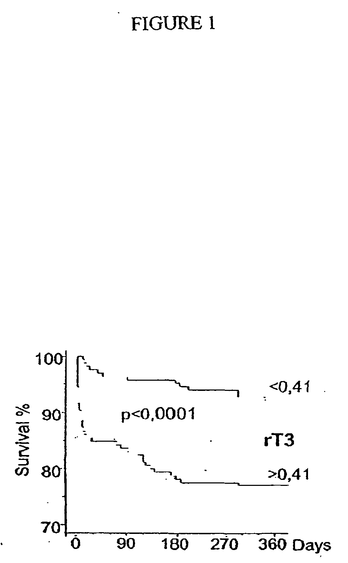

Figure 1 shows the percent of survival versus time. Thus the increased serum concentrations of rT₃ in patients with acute myocardial infarction is a new, not previously identified independent risk factor for death within the first year of the event.

### Example 2

### Experimental Details

### Anesthesia

All animals were fasted overnight and anesthesia was induced by intravenous sodium thiopental (Pentothal sodium 15-25 mg/kg). After intubation and ventilation by a ventilator (North American Dräger, Anesthesia and Ventilator, Model AVE-K, Serial No. 5033), anesthesia was maintained by 2% isoflurane (Isoflurane Vaporizer, R-Vapor, R-24045), and oxygen. ECG (Hewlett Packard Model No. 78346A). Oxygen saturation was monitored continuously. Preoperatively, all animals received Acepromazine maleate, 0.25-0.5 mg, I.M, lactated Ringer's solution was given intravenously (250 ml - 350 ml/hr) during the procedure.

### Methods

A 8.5Fr. catheter sheath was introduced into the right femoral artery and the side arm was connected to a fluid transducer for the continuous measurement of systemic arterial blood pressure. A 7Fr. Bipolar Multipurpose A-2 Electrode Catheter, 1 Lumelec^{™} (Lot no. 30395908, Catalog no. 528-724, Cordis, USA) tipped with pressure transducer with 2 side holes, 2 electrodes with an open end, 125 cm long, and 0.038 inch diameter, was advanced through the catheter sheath, and placed into the left ventricle. Continuous measurement of the systemic left ventricular pressure was obtained. Analog signals from the pressure transducers were obtained using an amplifier (PM-1000, CWE Inc., Ardmore, PA).

A 8.5Fr. catheter sheath was introduced into the right femoral vein and a 7Fr. MP A2, Multipurpose high flow catheter (catalog no. 527-742, Cordis, USA) with open end and side holes was placed into the right ventricle for T₃ₐ injections. Another 7Fr. catheter sheath was introduced into the left femoral artery and a 6Fr. pigtail catheter (catalog no. 527-654S, 110 cm, 155° angled) was placed into the descending aorta for the collection of aortic blood samples after T₃ₐ injections into the right ventricle. ECG (Hewlett-Packard, Model No. 78346A, Serial No. 2320A00522) was continuously monitored. A temperature-monitoring device (T. SIN, Japan, designed for YSI series 400 thermistors), connected to the data acquisition system, was introduced into the external ears of all the animals to continuously measure body temperature.

### Fibrillation

The two distal ends of the 7Fr. Bipolar Multipurpose A-2 Electrode Catheter were connected to a Transformer. The electrical system can deliver 15V, 20mA AC current through the pacing catheter system. The anesthesia was stopped. A venous blood sample from the right ventricle was obtained. Lid reflexes returned in several minutes.

The lid reflexes were checked by the finger method system and when frequent blinking of the lids was obtained, procedures for the ventricular fibrillation were carried out. 15V, 20ma AC were directly passed through the left ventricular myocardium via the pacing catheter to fibrillate the heart. The time period for the electrical induction of ventricular fibrillation was an average of 4-5 seconds. Occasionally, longer periods of between 5 and 15 seconds of electrical induction were needed to induce ventricular fibrillation.

### Cardiac Resuscitation using Thumper

After 4.5 minutes of untreated ventricular fibrillation and without any respiratory support, CPR was initiated using a Michigan Instruments THUMPER^{®} (Michigan Instruments, Grand Rapids, Ml, Model no. 1004, Serial no. 2252) set to generate an arterial peak pressure during compression of at least 60 mm Hg, simulating a palpable pulse generated by manual chest compression was carried out according to the American Heart Association instructions.

The force of compression necessary to achieve this baseline condition was recorded and not altered during subsequent provision of chest compressions. Ventilation was pressure limited (30 cm H₂O), providing 100% oxygen. The compression rate was set at 60 compressions/min with a compression/relaxation ratio of 1:1 and a compression/ventilation ratio of 5:1.

Data acquisition (10 minutes longer a file) was started at 4 minutes after ventricular fibrillation to cover the entire procedure after defibrillation and recovery. Two infusions of sodium bicarbonate (0.5 mEqv/kg) were given during THUMPER CPR within 2-minutes range to correct the base deficit.

Phase 1a was dedicated towards developing the basic methodology in the initial experiments, manual chest compression (CPR) and an internal defibrillator were employed. Induction of ventricular fibrillation and defibrillation were problematic with the internal electrodes because of the size mismatch: electrodes designed for humans being used in a smaller sized animal. In later experiments, the internal system was replaced with an LV pacing catheter connected to AC current via a step-down transformer (to induce ventricular fibrillation), and a new external, "hands-off" defibrillator. The manual chest compression was replaced with a THUMPER CPR system.

In the experiments, T₃ₐ was given 30 seconds before defibrillation. Preferably, T₃ₐ injection was given 60 to 90 seconds after defibrillation.

In Phase 1b, restoration of spontaneous circulation was achieved in 7 out of 8 animals with the changes to the protocol. One animal (T₃ #15) had an aortic dissection during the procedure, and was thus excluded from the study. Two animals had recovery of spontaneous circulation without T₃ₐ.

Further experimental chronic laboratory and human clinical studies are needed to determine efficacy of using this drug and new CPR techniques.

### Advanced Cardiac Life Support using T₃

At 6 minutes after inducing V-fib, defibrillatory shocks were administered according to the Advanced Cardiac Life Support algorithm, starting with an initial energy level of 200 joules, then increased to 300 joules, and if still not successful, to 360 joules. Lidocaine, atropine, and epinephrine were not used for cardiac resuscitation.

A Physio-Control Life Pack 9A system (Physio-Control Inc., Medtronic, US) was used to defibrillate the animals. Patient ECG cable (3-lead, AHA, Physio-Control PN 9-10418-02) was connected to all the animals for simultaneous synchronous or asynchronous defibrillation. QUICK-COMBO defibrillation cables (Physio-Control PN 806717) were used with EDGE SYSTEM^{™} therapy electrodes were attached to all animals. One electrode (+, black cable connector) was placed left lateral to the animal's sternum with the center of the electrode in the left mid-axillary line towards the apex of the myocardium. Another electrode (-, red cable connector) was placed at the apical aspect of the right lateral portion of the animal's chest in the right mid-axillary line.

200 joules of counter shock were administered to defibrillate the animals. In one animal, a further counter shock was needed. The THUMPER CPR was continued until spontaneous circulation was recovered. At 60 to 90 seconds after defibrillation, a bolus dose of T₃ₐ was injected into the right ventricle. The aortic pressure trace was momentarily set to zero to denote the time of the T₃ₐ injection. Thirty seconds later, a left ventricular blood sample was collected to determine the T₃ₐ blood level. Shortly after T₃ₐ injections (30 to 90 seconds), restoration of the spontaneous circulation was achieved in most instances.

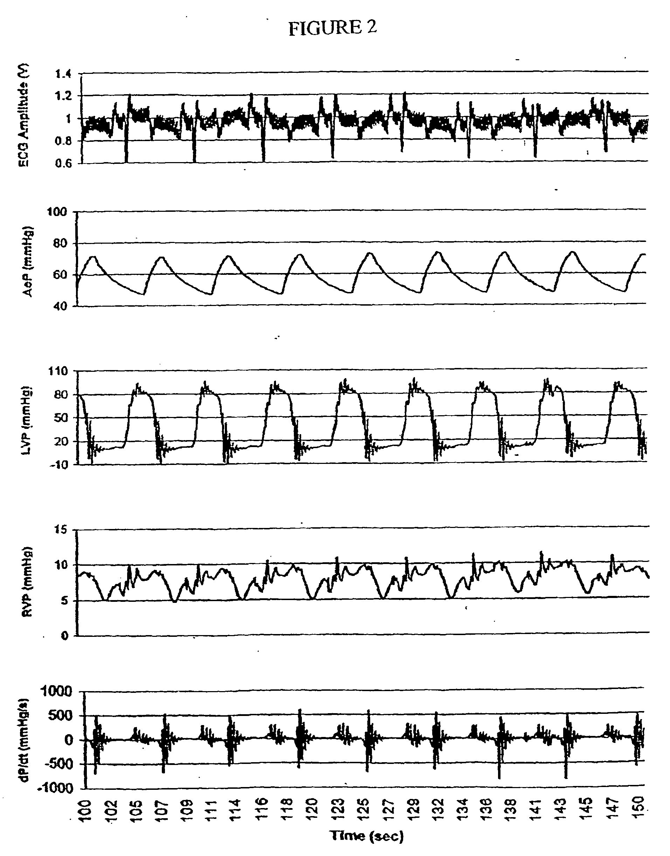

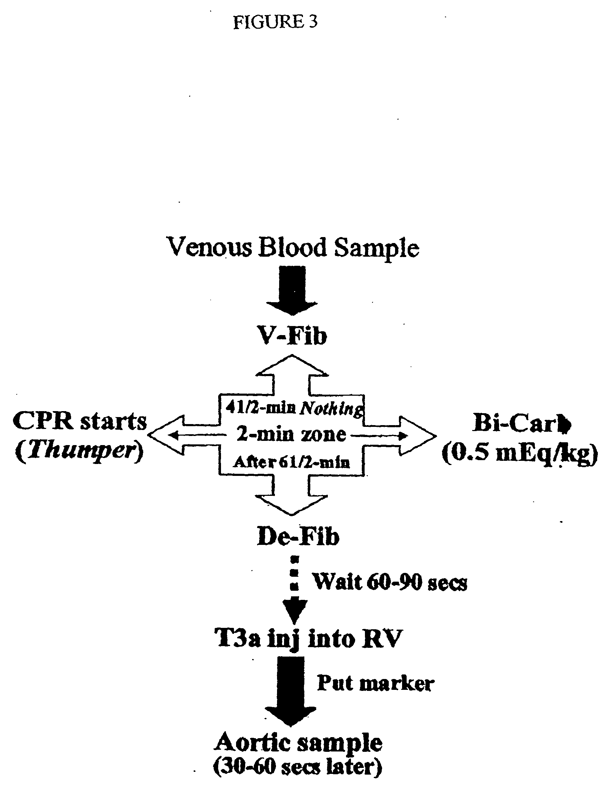

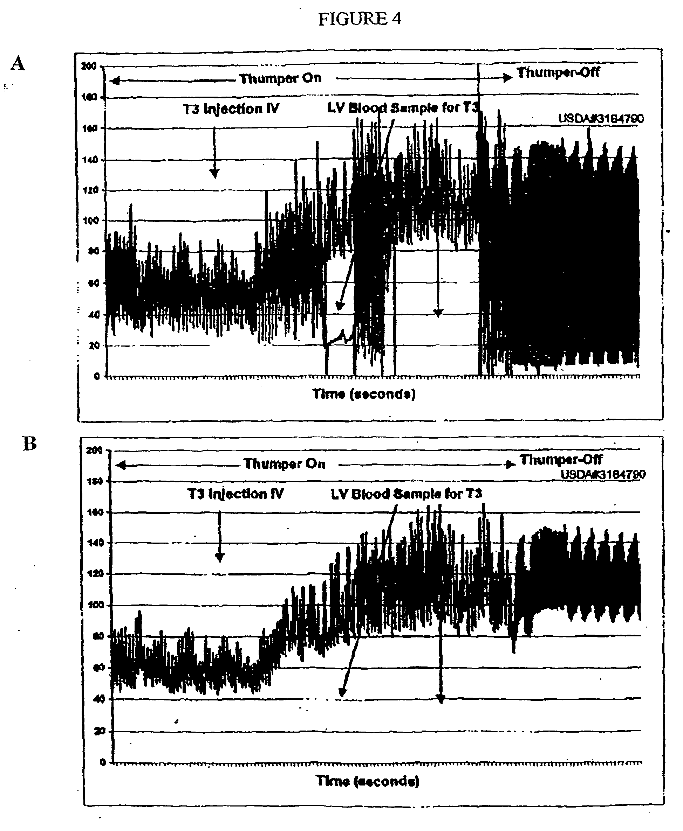

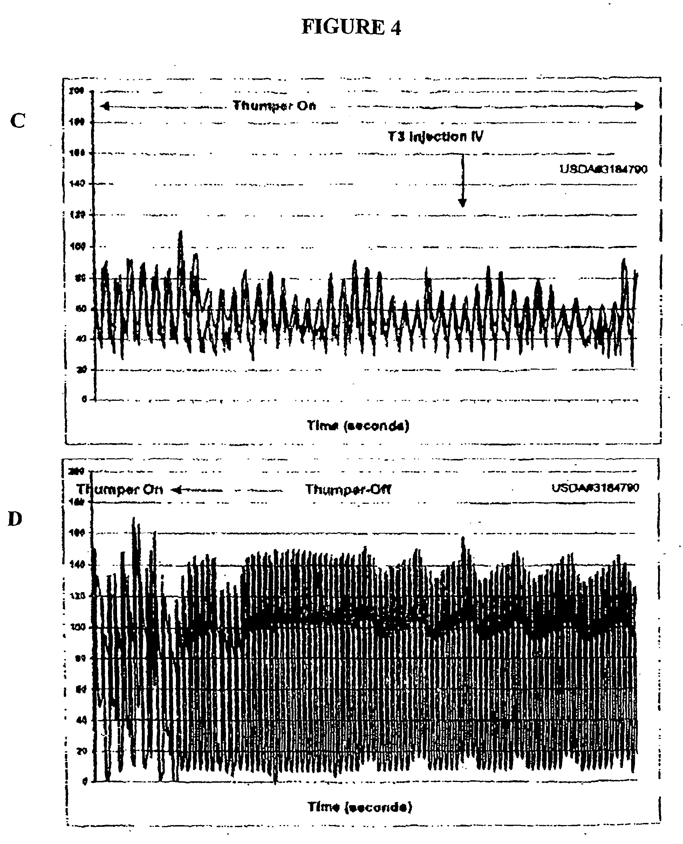

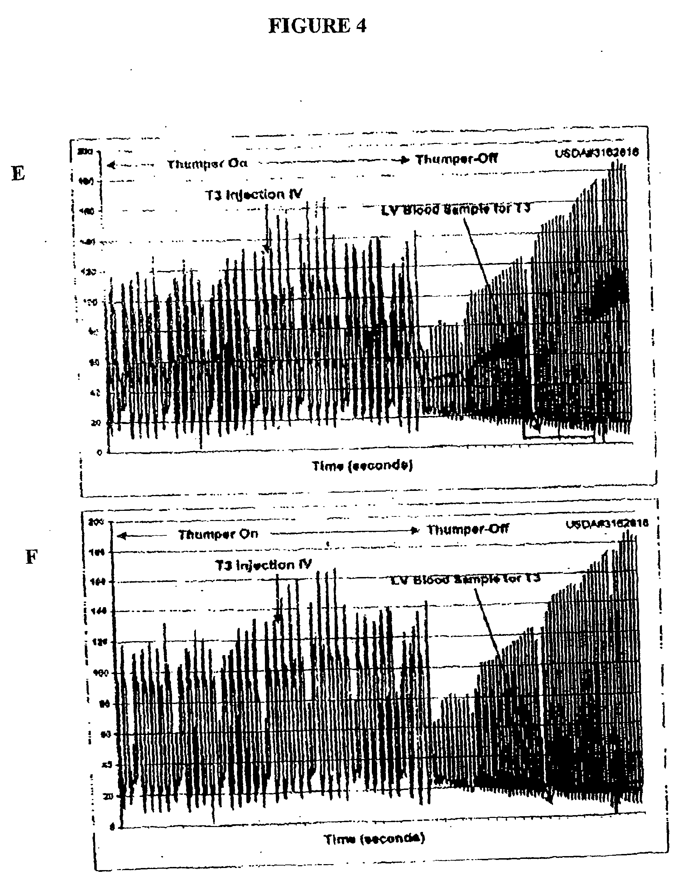

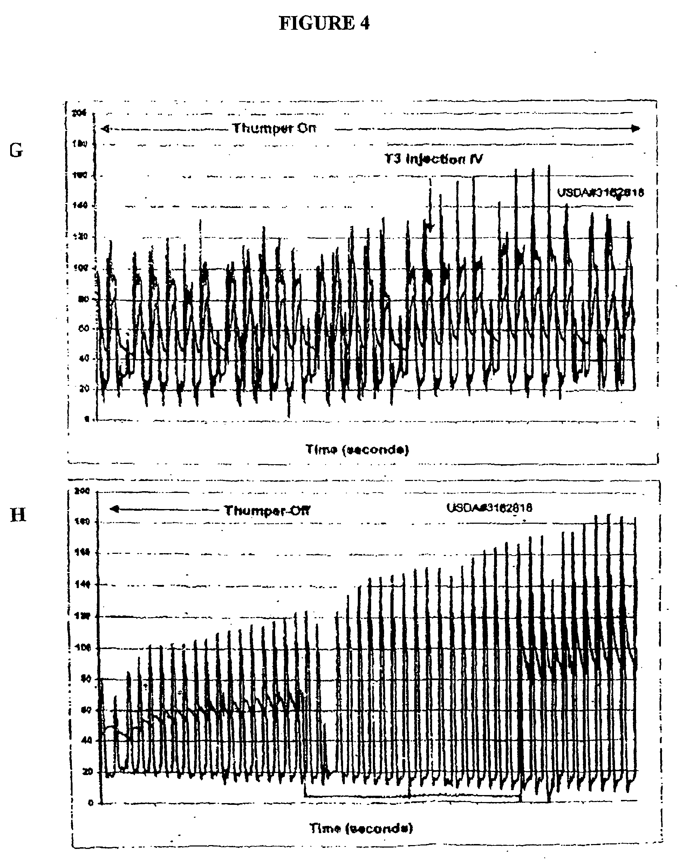

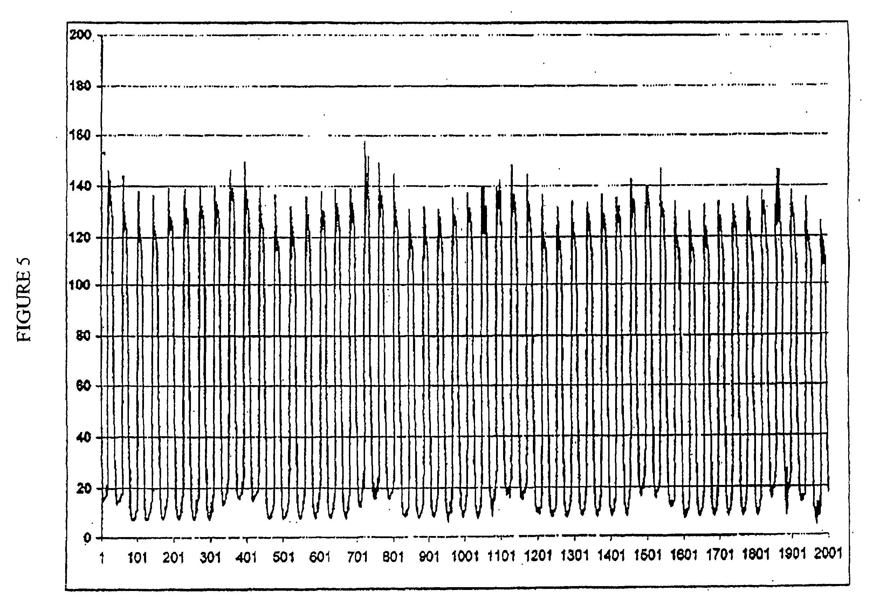

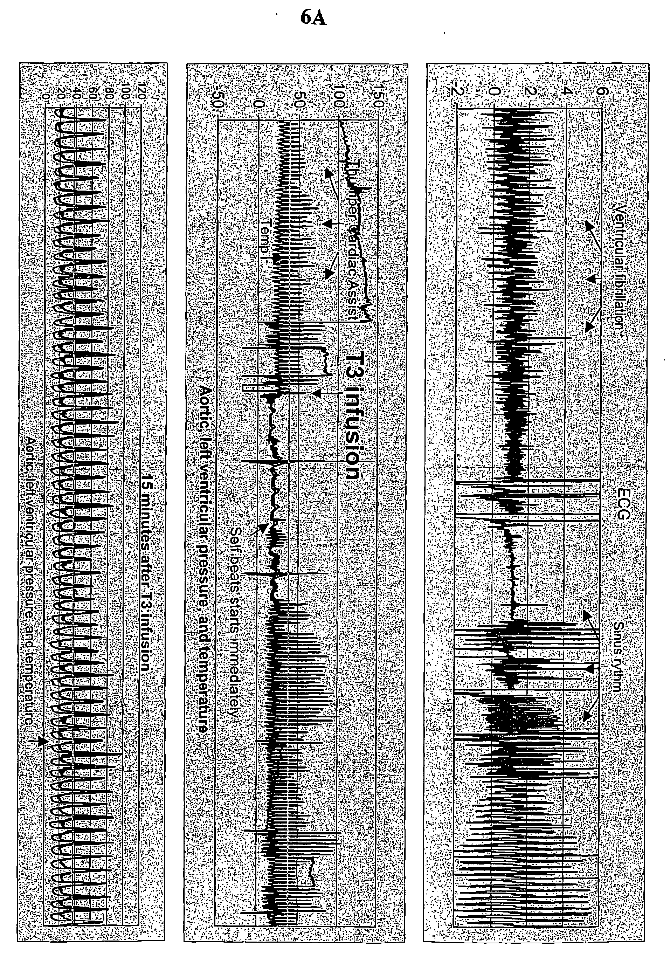

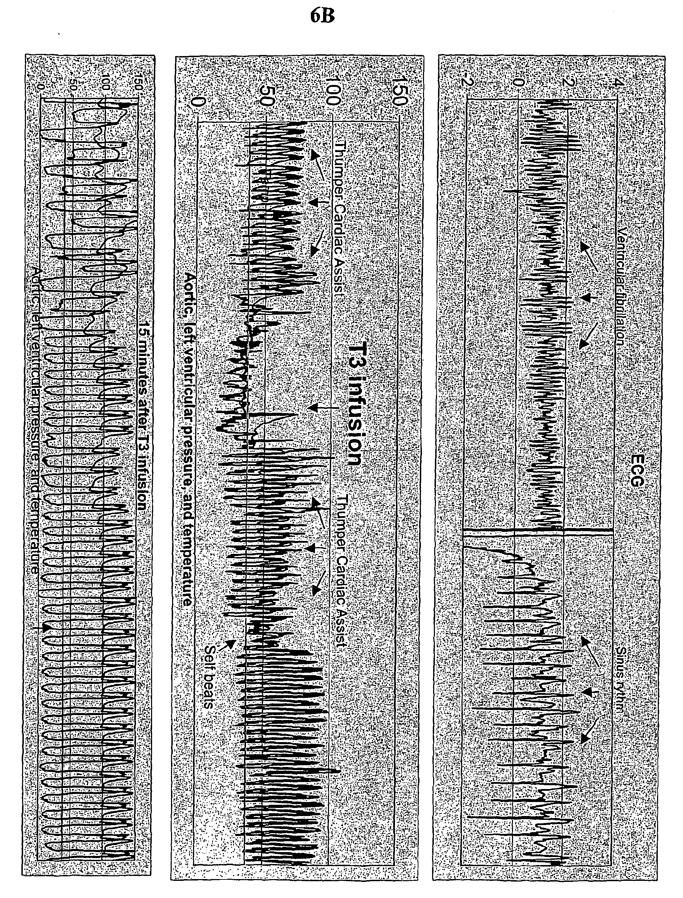

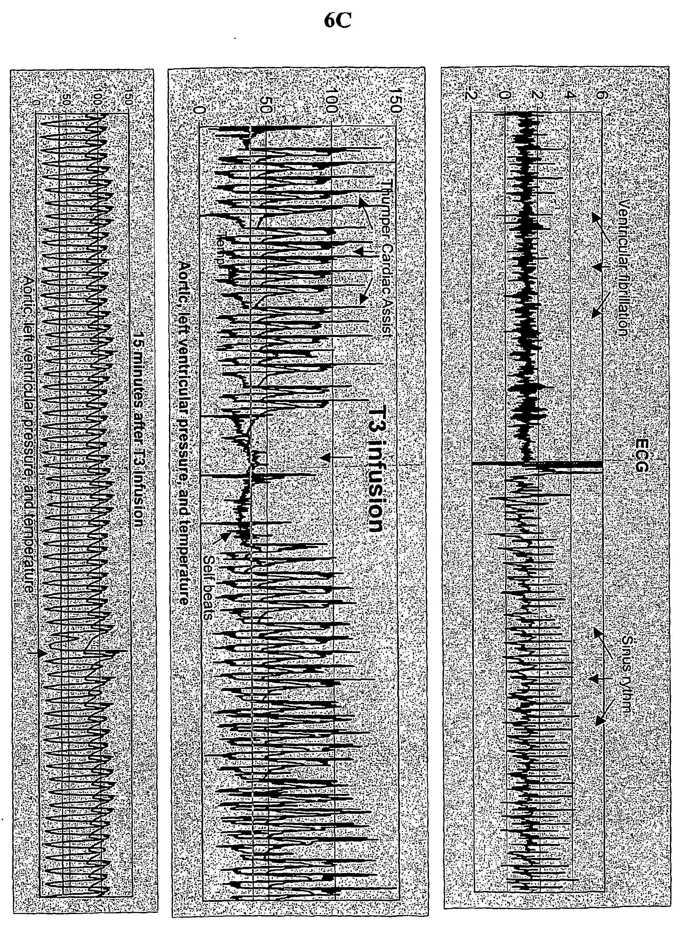

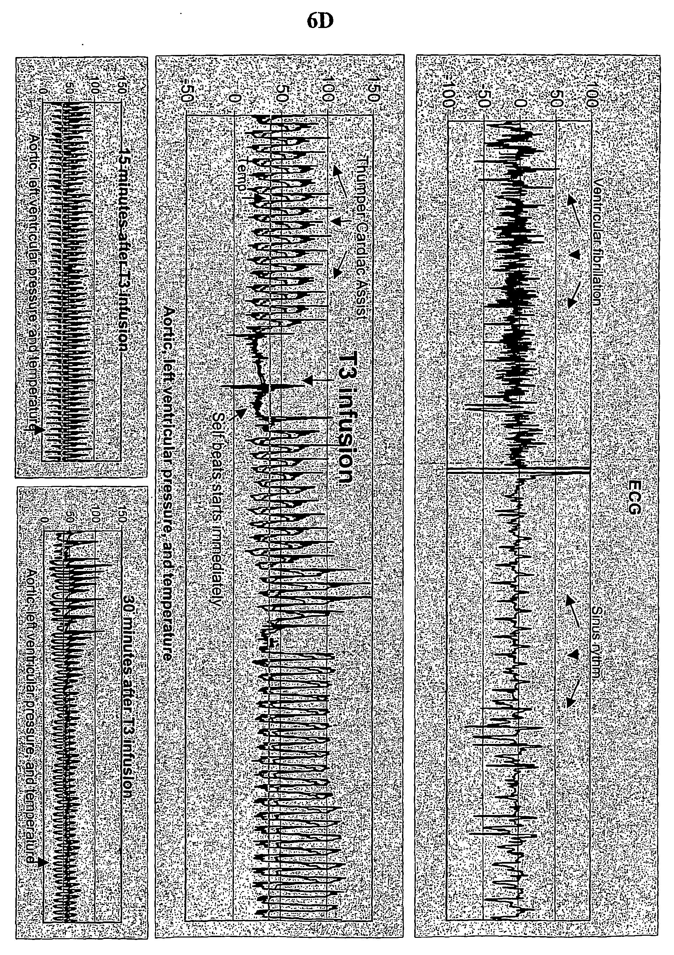

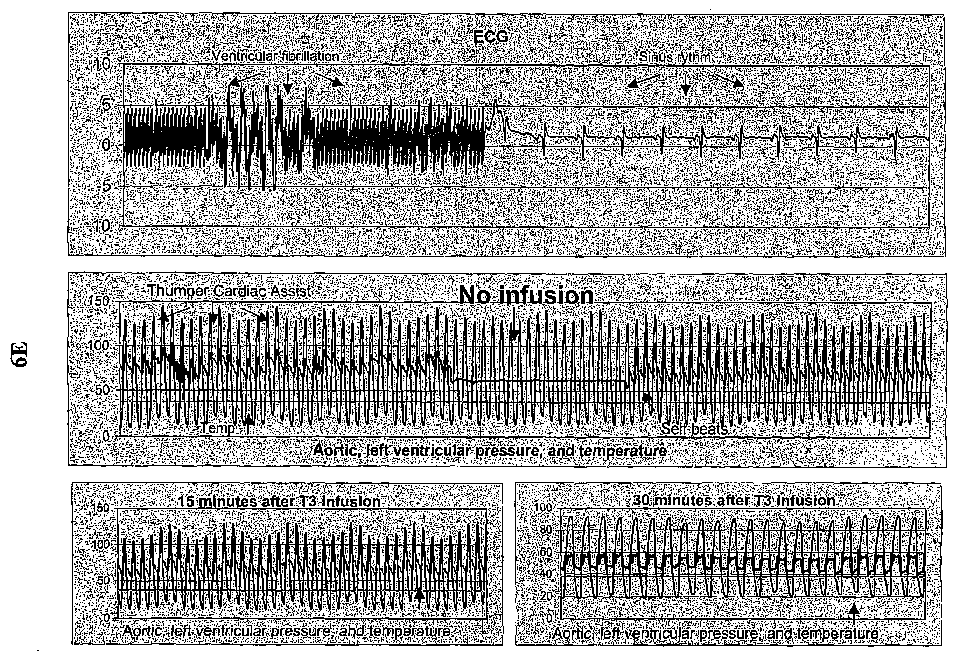

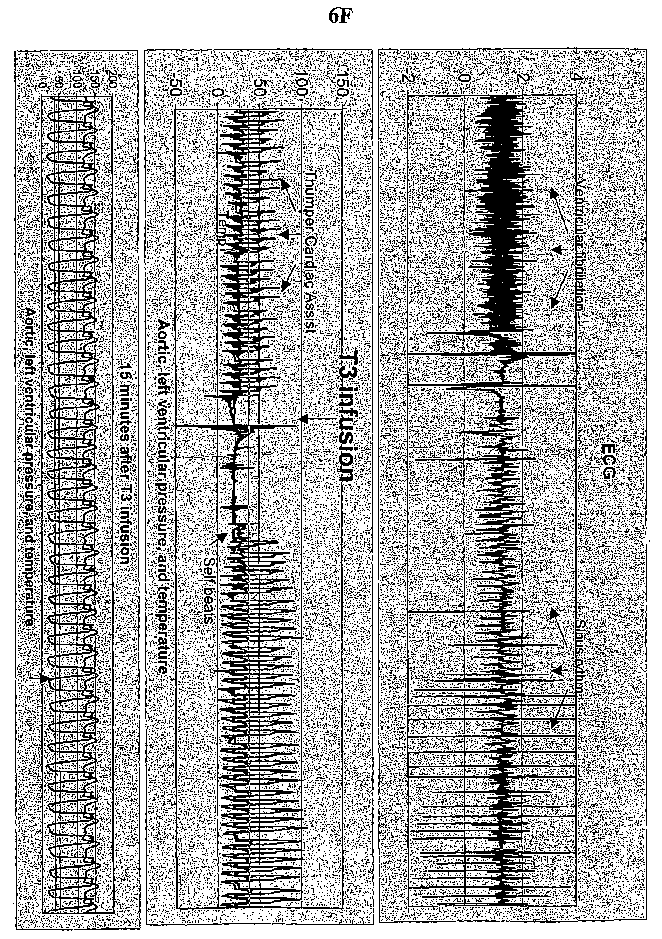

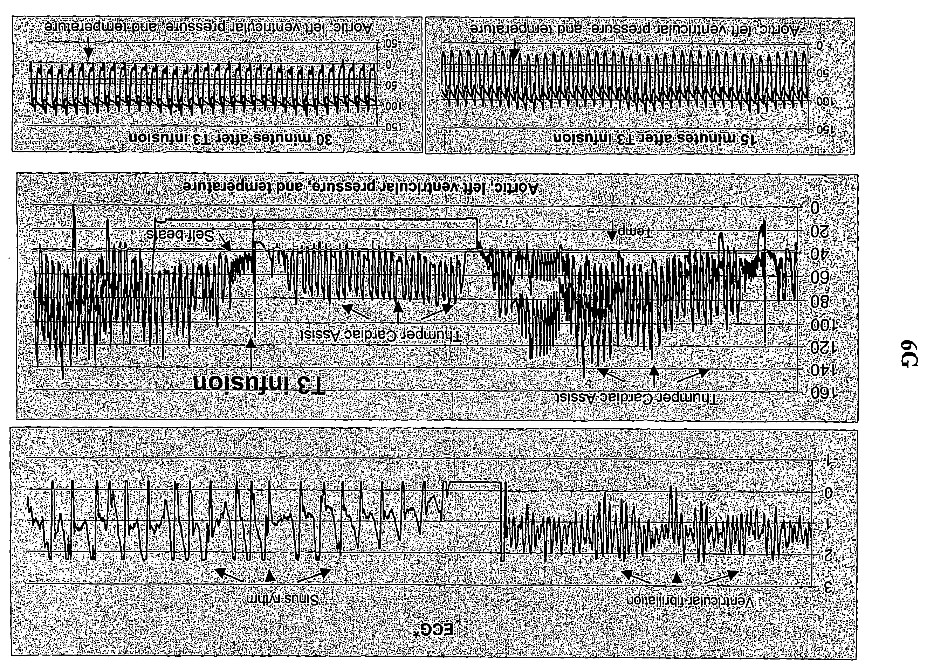

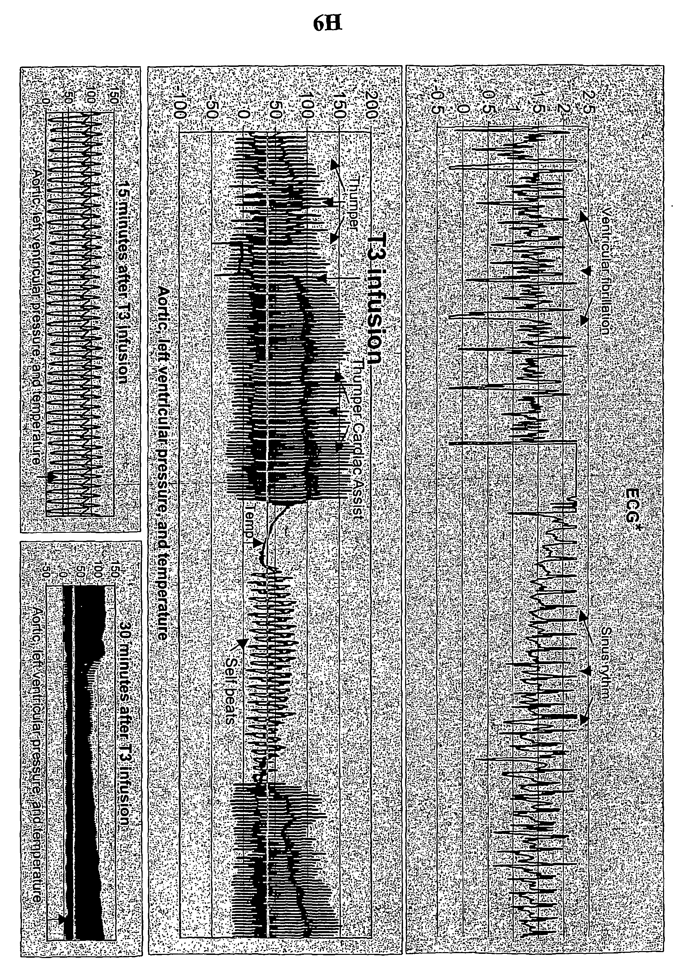

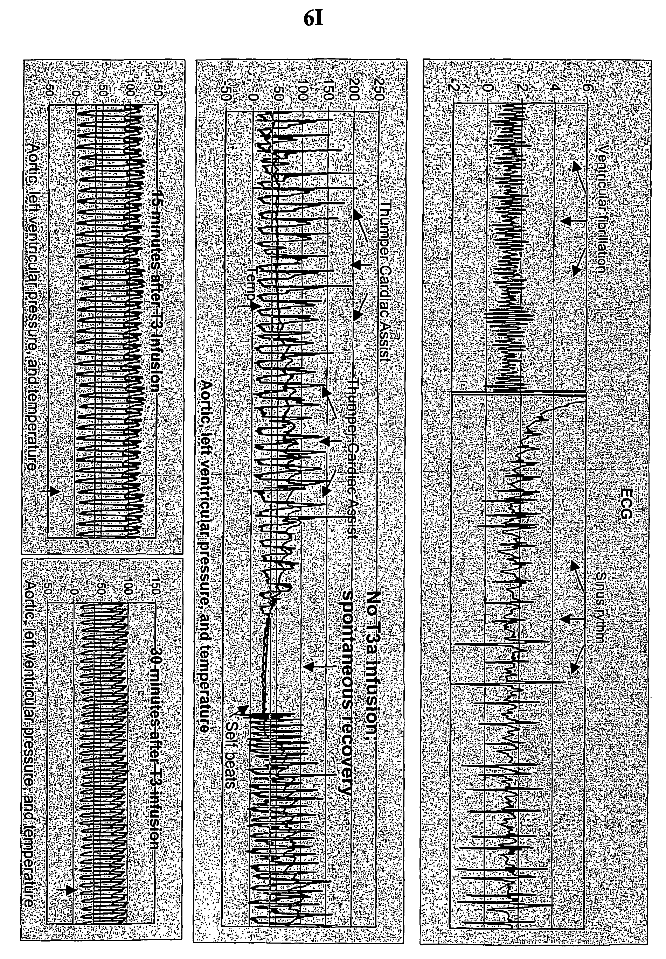

Restoration of the spontaneous circulation was defined as a pulsatile rhythm with a systolic arterial pressure of at least 60 mmHg. No further interventions or drugs were given. Lactated Ringer's Fluid infusion was maintained at about 10 mL/kg/hr. The animal was reconnected to the ventilator and after several minutes isoflurane anesthesia was restarted at a rate of 0.5%. Anesthesia was carefully maintained to avoid the cardiac decompensation. The animal was observed for another 30 minutes for any further changes in the arterial and left ventricular pressures. Another venous blood sample was taken after 15 minutes of ventricular fibrillation. Representative results are shown in Figures 2-6.

### Example 3

### Triiodothyronine-human serum albumin preparation (T₃ₐ)

100 *µ*g T₃/ml (1.5 x 10⁻⁴ M) was combined with a physiological concentration of 5% human serum albumin (HSA, 50 mg/ml, 762 *µ*M) at pH 7.2. The binding affinity between T₃ and albumin is low, the hormone-albumin complex dissociates rapidly. The examples provided herein show that the high capacity-low affinity binding complex is ideal in its ability to bind more than 1000 times normal serum T₃ concentrations, maintain the T₃ in solution at neutral pH and make T₃ rapidly available to tissues after intravenous administration.

In the body, albumin (66 kD) binds approximately 15-20% of the total serum T₃. The remaining T₃ is bound by other blood proteins including thyroxine-binding globulin (TBG) and transthyretin, so that 99% of the hormone in serum is protein-bound.

Binding characteristics of T₃ to albumin in phosphate-buffered saline at 37°C provide association constants of 1.0 x 10⁵ M⁻¹ at site 1 and 6.9 x 10³ M⁻¹ at sites 2-6. Gray (1979), Hormones in Blood, eds.; 3rd ed. Vol. 1. London: Academic, p. 576. As shown herein, T₃ₐ preparations contain approximately 90% albumin-bound T₃, which, when administered, dissociates rapidly. Preparation of the T₃ₐ composition: combination of 5% human serum albumin with a T₃/sodium hydroxide solution (10 mg T₃/ml 0.05 N NaOH) produces a T₃ₐ preparation which is a neutral pH 7.2 and is 100% soluble in solution.

### Example 4

### Studies of Cardiac Resuscitation with T₃ and Bioavailability of T₃ₐ Composition

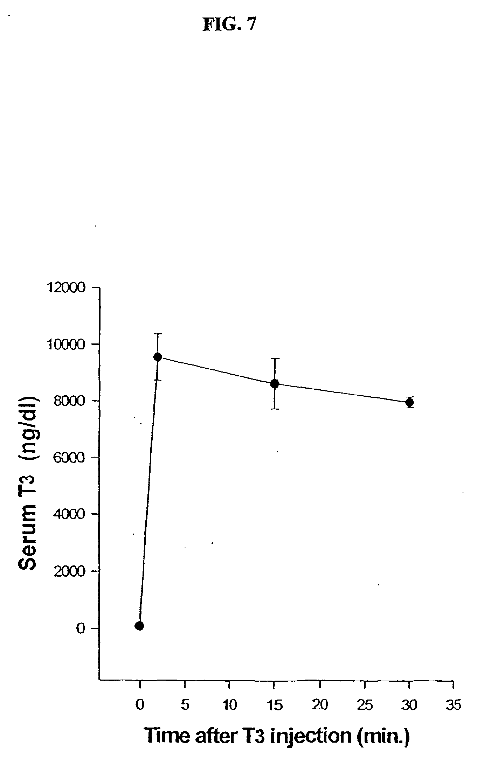

The T₃ preparation used in these studies was in the T₃ₐ formulation prepared as follows. (1) T₃ was dissolved in a physiological concentration of human serum albumin (HSA) (50 mg/ml), at a concentration of 0.10 mg /ml and pH 7.4. The samples were stored at room temperature, (2) T₃ₐ was injected as a bolus dose of 4 µg/Kg body weight approximately one minute after defibrillation was initiated and (3) a baseline serum T₃ value was obtained for each animal before ventricular fibrillation was induced. Subsequent blood samples were obtained from the arterial circulation at 0.5 to 1.5 minutes after the bolus injection, and also at 15 to 20 minutes and 30 minutes, respectively. The changes in serum T₃ concentration are shown in Figure 7, the serum total T₃ levels (ng/dl) as a function of time after injection.

The baseline serum T₃ level of 87 ± 6 ng/dl is within the physiological range for canines, and this was increased 100-fold one minute after bolus T₃ injection, and remained high over the 30 minute period of cardiac resuscitation.

### Example 5

### Immunoassay for T₃ₐ and the determination of hemodynamic parameters during 2-phase intervention with T₃ₐ drug in fibrillation and defibrillation procedures

Before sending the blood sample for immunoassay, in each experiment all the blood samples were collected in red-topped serum separator tubes. The tubes were centrifuged at 2500 rpm on a tabletop centrifuge for 10 minutes at 4°C to separate the cells from the serum. The blood samples were then stored and frozen at -4°C.

The results section was divided into Phases Ia (n=9) and Ib (n=8). The hemodynamic data were not consistently available in Phase Ia as there was no spontaneous or induced recovery except for 3 animals. Use of T₃ₐ was limited to only 3 dogs in this phase although epinephrine was used in 5 of them. This phase was dedicated towards the development of the Phase Ib parameters.

In Phase Ib, 2 animals showed spontaneous recovery. In one animal, the hemodynamic pressures were not stable and needed injection of epinephrine (0.1 mg/kg) to increase the reduced hemodynamic pressures. The other animal had spontaneous recovery after 4.5 minutes of ventricular fibrillation. The pressures were comparatively stable in this animal although lower than pre-VF hemodynamic data. In the same animal, we induced a 6-minute ventricular fibrillation and gave CPR for two minutes. The animal was defibrillated and given a T₃ₐ injection after 60 seconds. The animal was maintained on Thumper CPR for additional 1-2 minutes and was resuscitated completely. The animal was observed for another 30 minutes and the pressures were completely stable. The hemodynamic pressures were higher then the pro-VF hemodynamic data.

HVS-02 Fibrillator and Defibrillator were used in the tests. The operating instructions for the HVS-02 Fibrillator and Defibrillator can be found in the Manual for HVS-02 Fibrillator and Defibrillator. Tables 1 and 2 showed the hemodynamic parameters during 2-phase intervention with T₃ₐ in fibrillation and defibrillation procedures.

**Table 1**

| Phase Ib (Precise T₃ₐ working section) = 8 animals (test) | | | | | | | | |
|---|---|---|---|---|---|---|---|---|
| **Test No.** | **Time frame** | **S. AoP mmHg** | **D. AoP mmHg** | **S. LVP mmHg** | **Defib. Type** | **Recover** | **T₃ₐ use** | **Epi use** |
| 18 | Pre-VF | 113.8 | 95.5 | 118.7 | | | | |
| | VF+CPR (4.5m) | 44.5 | 39.6 | 70.1 | | | | |
| | After D-Fib+T₃ₐ | 86.4 | 62.6 | 107.5 | External | Yes | No | No |
| | Post T₃ₐ (15m) | 88.1 | 72.2 | 95.6 | | Yes | | No |
| 18 | Pre-VF | 92.5 | 80.5 | 100.1 | | | | |
| | VF+CPR (6.5m) | 44.5 | 32.1 | 72.1 | | | | |
| | After DFib+T₃ₐ | 103.3 | 80.7 | 109.6 | External | Yes | Yes | |
| | Post T₃ₐ (15m) | 102.1 | 80.5 | 107.2 | | Yes | | No |
| 17 | Pre-VF | 86.6 | 76.9 | 107.4 | | | | |
| | VF+CPR | 59.3 | 37.5 | 73.6 | | | | |
| | After D-Fib+T₃ₐ | 63.7 | 37.5 | 84.0 | External | Yes | Yes | |
| | Post T₃ₐ (15m) | 89.3 | 76.1 | 110.2 | | Yes | | No |
| 16 | Pre-VF | 83.1 | 65.5 | 89.2 | | | | |
| | VF+CPR | 81.1 | 42.3 | 84.1 | | | | |
| | After D-Fib+T₃ₐ | 89.6 | 63.1 | 102.3 | External | Yes | Yes | |
| | Post T₃ₐ (15m) | 134.1 | 111.1 | 138.8 | | Yes | | No |
| 15 | Pre-VF | 98.7 | 68.0 | | Aortic | | | |
| | VF-Defib | No** | | | Dissection* | | | |
| 14 | Pre-VF | 134.1 | 108.5 | 137.1 | | | | |
| | VF+CPR | 60.1 | 35.5 | 70.2 | | | | |
| | After D-Fib+T₃ₐ | 60.2 | 41.3 | 80.3 | External | Yes | Yes | |
| | Post T₃ₐ (15m) | 93.7 | 76.7 | 99.3 | | Yes | | No |
| 13 | Pre-VF | 80.7 | 68.0 | 89.9 | | | | |
| | VF+CPR | 80.6 | 64.5 | 87.2 | | | | |
| | After D-Fib+ | 56.7 | 45 | 80.0 | External | Yes | No | |
| | Post -- (15m) | 82.7 | 64.0 | 103.0 | | Yes | | Yes |
| 12 | Pre-VF . | 108.3 | 71.2 | 110.5 | | | | |
| | VF+CPR | 58.4 | 40.5 | 77.5 | | | | |
| | After D-Fib+T₃ₐ | 62.3 | 34.9 | 80.2 | External | Yes | Yes | |
| | Post T₃ₐ (15m) | 81.4 | 45.5 | 115.6 | | Yes | | No |
| 11 | Pre-VF | 106.7 | 78.4 | 118.5 | | | | |
| | VF+CPR | 52.3 | 32.1 | 80.1 | | | | |
| | After D-Fib+ T₃ₐ | 58.8 | 34.4 | 88.9 | External | Yes | Yes | |
| | Post T₃ₐ (15m) | 117.5 | 108.1 | 85.5 | | Yes | | No |
| 10 | Pre-VF | 134.1 | 108.5 | 137.1 | | | | |
| | VF+CPR | 81.1 | 42.3 | 84.1 | | | | |
| | After D-Fib+ T₃ₐ | 89.6 | 63.1 | 102.3 | External | Yes | Yes | No |
| | Post T₃ₐ (15m) | 134.1 | 111.1 | 138.8 | | Yes | | No |

| | | | | | | | | |
|---|---|---|---|---|---|---|---|---|
| * Aortic dissection occurred during blind manipulation of the LV pacing catheter. | | | | | | | | |

In Phase Ib, restoration of spontaneous circulation was achieved in 7 out of the 8 animalstested. In Table 1, one animal (T₃ #15) had an aortic dissection and was thus precluded from the procedure and two animals had recovery of spontaneous circulation without T₃ₐ. Moreover, in one of these eight animals, the circulation was unstable and required epinephrine and external compression to maintain the circulation. Furthermore, in one animal, the period of non-support before applying CPR was extended to 6 minutes and this animal (T₃ #18) had full recovery of the circulation with T₃ₐ. In this phase, i.e. Phase Ib, T₃ₐ was clearly effective in restoration of spontaneous cardiopulmonary circulation in the preliminary study.

**Table 2**

| Phase Ia (Methodology section) = 9 animals (test) | | | | | | | | |
|---|---|---|---|---|---|---|---|---|
| **Test No.** | **Time frame** | **S. AoP mmHg** | **D. AoP mmHg** | **S.LVP mmHg** | **Defib. Type** | **Recover** | **T₃ₐ Use** | **Epi use** |
| 9 | Pre-VF | 90 | 62.5 | 95.5 | | | | |
| | VF+CPR* | 55 | 32.0 | 60.0 | | | | |
| | After D-Fib+T₃ₐ | | | | External | No | Yes | No |
| | Post T₃ₐ (15m) | | | | | No | | No |
| 8 | Pre-VF | 96.0 | 70.1 | 98.5 | | | | |
| | VF+CPR | 45 | 25.5 | 50 | | | | |
| | After D-Fib+T₃ₐ | 60 | 35.6 | 68.7 | External | Yes | Yes | |
| | Post T₃ₐ (15m) | 80.1 | 60.3 | 85.5 | | Yes | | No |
| 7 | Pre-VF | 122.3 | 97.7 | 133.3 | | | | |
| | VF+CPR | | | | | | | |
| | After D-Fib+T₃ₐ | | | | External | No | Yes | |
| | Post T₃ₐ (15m) | | | | ** | No | | No |
| 6 | Pre-VF | 90 | 60 | 98 | | | | |
| | VF+CPR*** | 60 | 32 | 68 | | | | |
| | After D-Fib+T₃ₐ | 77 | 50 | 85 | External | Yes | Yes | Yes |
| | Post T₃ₐ (15m) | 93.7 | 77 | 99 | *** | Yes | | Yes |
| 5 | Pre-VF | 98 | 68 | See | | | | |
| | VF+CPR | **** | | note | | | | |
| | After D-Fib+ | | | | Internal^{†} | No | No | Yes |
| | Post -- (15m) | | | | | No | | Yes |
| 4 | Pre-VF | 80 | 65 | 89 | | | | |
| | VF+CPR | ‡ | | | | | | |
| | After D-Fib+ | | | | Internal | No | No | Yes |
| | Post -- (15m) | | | | | No | | Yes |
| 3 | Pre-VF | 86 | 60 | 90 | | | | |
| | VF+CPR | ***** | | | | | | |
| | After D-Fib+ | | | | Internal | Yes | No | Yes |
| | Post T₃ (15m) | | | | External | No | | |
| 2 | Pre-VF | 89 | 65 | 96 | | | | |
| | VF+CPR | 60 | 48 | 66 | ****** | | | |
| | After D-Fib+ | 65 | 50 | 84.0 | Internal^{ξ} | Yes | No | No |
| | Post -- (15m) | 89.3 | 76.1 | 110.2 | | Yes | | Yes |
| 1 | Pre-VF | 80 | 65 | 90 | | | | |
| | VF+CPR | Ψ | | | | | | |
| | After D-Fib+ | | | | Internal^{Ψ} | | No | No |
| | Post -- (15m) | | | | | | | |

| | | | | | | | | |
|---|---|---|---|---|---|---|---|---|
| • * Thumper did not work out in this large dog and CPR was given by manual chest compression • ** Defibrillation was not possible and it appeared that defibrillator was not working properly • *** Fibrillation and defibrillation procedures were tested 3 times in this animal to test for the first time, the external defibrillator. After a 2 minute wait, the animal was given 1 mg of epinephrine plus sodium bicarbonate and CPR, to defibrillate the animal. The same experiments were done with a 3-minute interval, and this time, T₃ₐ was also used after epinephrine and the dog was defibrillated with normal pressure development. We further performed the same experiments with 4-minute interval using T₃ₐ. The results were immediate and the animal developed pressure very rapidly. The dog was fully recovered. • ****^{†} The electrodes for the internal defibrillation procedure were partially moved, especially the one with the superior vena cava. Defibrillation was not immediately achieved. Epinephrine and lidocaine injections were given. Finally the animal was defibrillated (50 joules) although there was not any generated pressure development. • ‡ Animal was fibrillated and defibrillation was not achieved by 920 volts, 14 ms, resistance 70 ohms, and 49.2 joules of shock. No blood pressure recovery was observed. The animal was defibrillated 3-4 times with an injection of 1 mL of epinephrine to achieve defibrillation. However, there was no spontaneous recovery. Cardiac massage was given compressing the heart directly by opening the chest. The study on this animal ended here. • ***** Fibrillation was for 3 minutes. After defibrillation; but there was no pulse. The animal went into VF again and was defibrillated. There was low pressure and the external defibrillator was applied. Full function was not recovered. Pentobarbital anesthesia was used for this study. • ******^{ξ}The animal was defibrillated several times, one at 2 minutes. The animal fully recovered without any drug. At 3 minutes, epinephrine was required although it needed 3 shocks. The animal was fibrillated, but after another 3 minutes, could not be recovered. • ^{ψ} The internal electrodes moved several times during fibrillation procedure due to position change of the dog. The defibrillation procedure was unsuccessful. | | | | | | | | |

Phase Ia was dedicated towards developing the basic methodology. Restoration of circulation was achieved in 3 animals out of 7 using manual chest compression (CPR). In 3 animals, the hemodynamic data after defibrillation were less than the pre-fibrillatory data. It was found that induction of ventricular fibrillation (VF) was problematic with the internal electrodes and defibrillation was problematic because of the size mismatch, i.e. electrodes designed for humans being used in a smaller sized animal. In the experiments, internal defibrillator was used for the preliminary experiments and T₃ₐ was given just before defibrillation (30 seconds). Moreover, THUMPER CPR was used in the last 2 dogs instead of manual CPR. Thumper CPR worked well and it was used for the subsequent T₃ₐ experiments of Phase Ib. Manual chest compression was maintained for another 5 minutes after defibrillation and T₃ₐ or epinephrine injections. Bolus epinephrine injection (100 µg/kg) was used in 5 random dogs.

### Example 6

### Studies to determine the stability of T₃ₐ, compared with T₃ alone

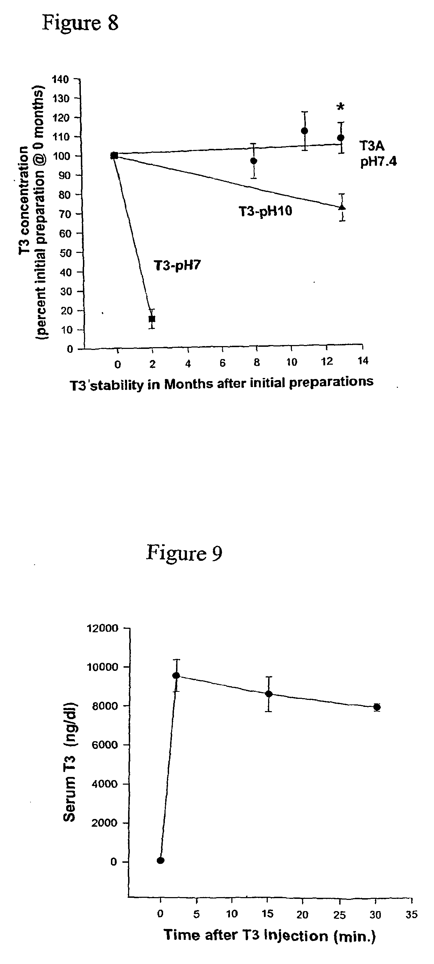

The triiodo-L-thyronine preparation used in these studies was in the T₃ₐ formulation prepared as follows. T₃ was dissolved in a physiological concentration of human serum albumin (HSA) (50 mg/ml), at a concentration of 0.10 mg / ml at pH 7.4. The samples were stored at room temperature for specific lengths of time as indicated in Figure 8.

T₃ was dissolved in 0.05N NaOH/saline and the pH adjusted to pH 7 or pH 10 to provide two other preparations of T₃ at a concentration of 0.10 mg/ml. These two preparations were stored under similar conditions as T₃ₐ and are shown in Figure 8 as T₃-pH 10 and T₃-pH 7. The total T₃ concentration in all the stock T₃ preparations were determined when initially made and at the times indicated using the radioimmunoassay described herein.

The results presented in Figure 8 show the following. The T₃ concentration in the T₃ₐ formulation remained unchanged from the original preparation for over 13 months of analysis. The concentration of T₃ in saline solution at pH 10 was decreased to 71 ± 7% of the original preparation after 13 months of storage at room temperature. The concentration of T₃ in saline solution at pH 7 was decreased to 14 ± 5% of the original preparation when measured following 2 months of storage at room temperature. The T₃ₐ stored at 37°C for 13 months retained 87 ± 5% of the initial T₃ concentration.

### Example 7

### Studies to Determine the Pharmacokinetics of T₃ₐ in vivo The half-life and stability of T₃ in serum after a single bolus dose

Figure 9 shows serum T₃ levels in the dog model of cardiac resuscitation in which dogs were given 4 µg T₃/kg body weight immediately after fibrillation and cardiac arrest. The desired effect was seen, that is the T₃ dosing in resuscitation as a requirement for an immediate effect of T₃ (within 30 min.) on the heart followed by rapid degradation of T₃ from serum.

The results showed that the serum T₃ levels increased to greater than 9000 ng/dl within 2 minutes of drug administration. These serum T₃ levels were maintained at this high level for 30 minutes.

### Pharmacokinetic studies in a rodent model

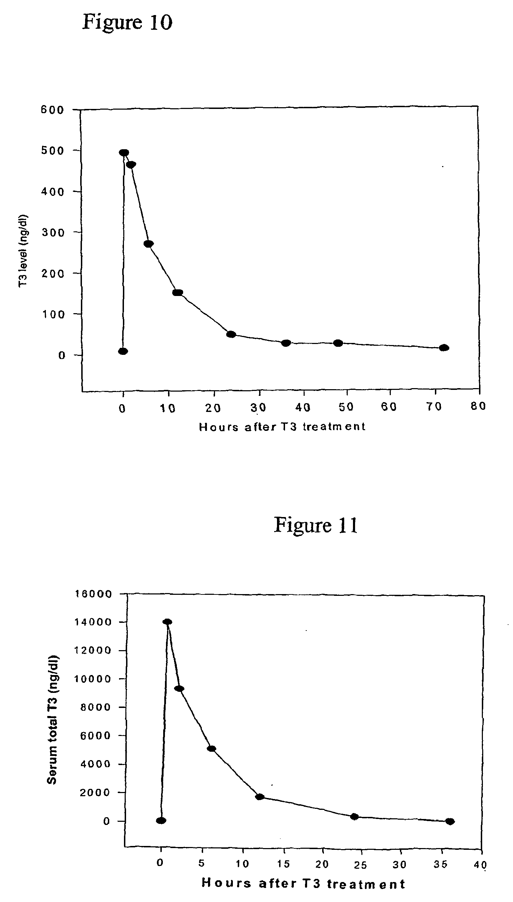

To further study the degradation or decrease of serum T₃ following a bolus dose of drug, the following studies were performed. Rats were injected intramuscularly with two doses of T₃: 5 µg/Kg and 100 µg/Kg body wt. Blood samples were collected over a 72 hour period and total T₃ was measured in serum by radioimmunoassay as described herein. The results are shown in Figures 10 and 11.

Figures 10 and 11 show the following. The rapid increase in serum T₃ levels was proportional to the bolus dose administered. Peak values were obtained within 30 minutes of drug administration. Within 2 hours of drug injection, the serum T₃ levels decreased significantly to 90% and 64% of the peak values for the low and high doses, respectively. By 24 hours after injection of the drug, the serum T₃ levels had decreased to 10% of peak values, and were within normal physiological range. The half-life of T₃ in serum after injection of either 5 µg/kg or 100 µg T3/kg body weight was identical.

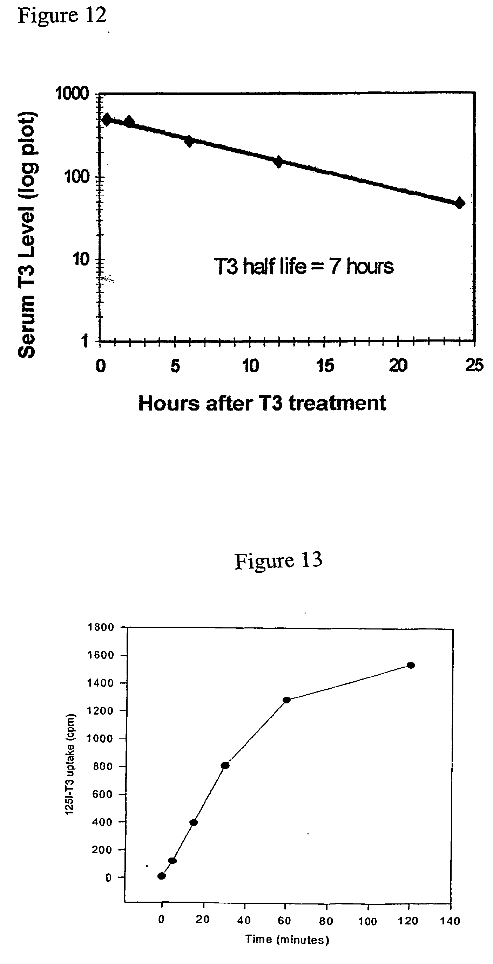

Figure 12 shows the log plot of T₃ in serum over a time period calculated T₃ half-life of seven (7) hours.

### Example 8

### Studies to determine uptake of T₃ into the cardiac myocyte:

In order to understand the potential biological benefit of T₃ on the heart following cardiopulmonary resuscitation, it is important to document the uptake of the drug into the cardiac myocyte within the time frame used for the resuscitation procedure.

Studies were designed to measure the rate of uptake of T₃ into the heart using purified cultured cardiac myocytes. The time course of T₃ uptake into the cardiac myocyte was followed by treating the cells with radio-labeled T₃ (¹²⁵I-T₃).

The results showed that T₃ is detected in the nucleus of the cell within 5 minutes of exposure to T₃ at a dose of 10⁻⁸ M (serum levels of T₃ equivalent to 650 ng/dl), and that the T₃ uptake reached saturation by approximately 2 hours. These results are illustrated in Figure 13.

## Claims

1. A composition comprising between 0.01 mg/ml and 1.0 mg/ml T₃, serum albumin and water wherein the stability of T₃ is increased.

2. A composition according to claim 1, wherein the T₃ has a half-life of at least five days at a temperature range of about - 30°C to 70°C.

3. A composition according to claim 1, wherein the T₃ has a half-life of:
a) at least one month;
b) at least three months;
c) at least six months; and/or
d) at least twelve months.

4. A composition according to any one of the preceding claims, wherein the ratio of T₃ and the serum albumin is:
a) between about 0.001 and 0.1; and/or
b) between 0.002 and 0.05.

5. A composition according to any one of the preceding claims, wherein the T₃ has a concentration of:
a) between 0.02 mg/ml and 0.08 mg/ml;
b) between 0.1 mg/ml and 0.5 mg/ml; or
c) 0.1 mg/ml.

6. A composition according to any one of the preceding claims, wherein the pH range is:
a) 2.5 to 11.5;
b) 4.0 to 10;
c) 6.0 to 8.0; or
d) 6.5 to 7.5.

7. A composition according to any one of the preceding claims which further comprises a pharmaceutically acceptable excipient.

8. A composition according to claim 7, suitable for use in:
a) intravenous administration;
b) direct cardiac administration;
c) parenteral administration; or
d) mucosal administration.

9. A composition according to claim 8, wherein the mucosal administration is selected from the group consisting of intranasal, by-inhalation and buccal.

10. A composition according to claim 9, wherein the T₃ has a half-life of at least two weeks.

11. A composition according to any one of the preceding claims, wherein the serum albumin is human serum albumin or bovine serum albumin.

12. Use of a composition according to any one of the preceding claims for the manufacture of a medicament for treating a patient with cardiac arrest, or with cardiac electrical standstill, to restore effective cardiac function.

13. Use according to claim 12 wherein:
a) the cardiac arrest is caused by electromechanical dissociation; or
b) the cardiac electrical standstill is caused by a disease.

14. Use according to claim 12 or 13, wherein the composition is administered:
a) by direct injection to a heart cavity of the patient;
b) by direct parenteral injection into a central venous line of the patient;
c) by parenteral injection or parenteral intravenous injection;
d) directly to the pulmonary system of the patient;
e) directly to the pulmonary system by direct endotracheal injection;
f) directly to the pulmonary system by infusion through a respiratory airway of the patient;
g) via intravenous drip; or
h) via mucosal delivery.

15. Use according to any one of claims 12 to 14, wherein the composition is administered in at least one rapid bolus injection.

16. Use according to any one of claims 12 to 15, wherein the composition is administered at:
a) between 0.1 and 20 µg T₃ per kg of body weight;
b) between 0.2 and 10 µg T₃ per kg of body weight;
c) between 0.3 and 5 µg T₃ per kg of body weight; and/or
d) 100 µg T₃ per kg of body weight.

## Patentansprüche

1. Zusammensetzung, umfassend 0,01 mg/ml bis 1,0 mg/ml T₃, Serumalbumin und Wasser, wobei die Stabilität von T₃ erhöht ist.

2. Zusammensetzung nach Anspruch 1, wobei T₃ eine Halbwertszeit von mindestens 5 Tagen im Temperaturbereich von etwa -30 °C bis 70 °C aufweist.

3. Zusammensetzung nach Anspruch 1, wobei das T₃ eine Halbwertszeit von
a) mindestens 1 Monat;
b) mindestens 3 Monate;
c) mindestens 6 Monate; und/oder
d) mindestens 12 Monate aufweist.

4. Zusammensetzung nach einem der vorstehenden Ansprüche, wobei das Verhältnis von T₃ und Serumalbumin
a) etwa 0,01 bis 0,1; und/oder
b) 0,002 bis 0,05
beträgt.

5. Zusammensetzung nach einem der vorstehenden Ansprüche, wobei das T₃ eine Konzentration von
a) 0,02 mg/ml bis 0,08 mg/ml;
b) 0,1 mg/ml bis 0,5 mg/ml; oder
c) 0,1 mg/ml
aufweist.

6. Zusammensetzung nach einem der vorstehenden Ansprüche, wobei der pH-Bereich
a) 2,5 bis 11,5;
b) 4,0 bis 10;
c) 6,0 bis 8,0; oder
d) 6,5 bis 7,5 beträgt.

7. Zusammensetzung nach einem der vorstehenden Ansprüche, die ferner ein pharmakologisch verträgliches Exzipiens enthält.

8. Zusammensetzung nach Anspruch 7, geeignet zur Verwendung für die
a) intravenöse Verabreichung;
b) direkte Verabreichung ins Herz;
c) parenterale Verabreichung; oder
d) mukosale Verabreichung.

9. Zusammensetzung nach Anspruch 8, wobei die mukosale Verabreichung aus der Gruppe intranasale, Inhalations- und bukkale Verabreichung ausgewählt wird.

10. Zusammensetzung nach Anspruch 9, wobei das T₃ eine Halbwertszeit von mindestens 2 Wochen aufweist.

11. Zusammensetzung nach einem der vorstehenden Ansprüche, wobei es sich beim Serumalbumin um Humanserumalbumin oder Rinderserumalbumin handelt.

12. Verwendung einer Zusammensetzung nach einem der vorstehenden Ansprüche zur Herstellung eines Arzneimittels zur Behandlung eines Patienten mit Herzstillstand oder mit elektrischem Herzstillstand zur Wiederherstellung einer wirksamen Herzfunktion.

13. Verwendung nach Anspruch 12, wobei
a) der Herzstillstand durch elektromechanische Dissoziation verursacht wird; oder
b) der elektrische Herzstillstand durch eine Krankheit verursacht wird.

14. Verwendung nach Anspruch 12 oder 13, wobei die Zusammensetzung verabreicht wird:
a) durch direkte Injektion in einen Herzhohlraum des Patienten;
b) durch direkte parenterale Injektion in eine zentrale Venenleitung des Patienten;
c) durch parenterale Injektion oder durch parenterale intravenöse Injektion;
d) direkt in das pulmonale System des Patienten;
e) direkt in das pulmonale System durch direkte endotracheale Injektion;
f) direkt in das pulmonale System durch Infusion über einen Luftweg des Patienten;
g) über intravenöse Tropfinfusion; oder
h) über eine mukosale Abgabe.

15. Verwendung nach einem der Ansprüche 12 bis 14, wobei die Zusammensetzung in mindestens einer raschen Bolus-Injektion verabreicht wird.

16. Verwendung nach einem der Ansprüche 12 bis 15, wobei die Zusammensetzung in einer Menge von:
a) 0,1 bis 20 µg T₃ pro kg Körpergewicht;
b) 0,2 bis 10 µg T₃ pro kg Körpergewicht;
c) 0,3 bis 5 µg T₃ pro kg Körpergewicht; und/oder
d) 100 µg T₃ pro kg Körpergewicht
verabreicht wird.

## Revendications

1. Composition qui contient de 0,01 à 1,0 mg/ml d'hormone T₃, de la sérumalbumine et de l'eau, et dans laquelle la stabilité de l'hormone T₃ est renforcée.

2. Composition conforme à la revendication 1, dans laquelle l'hormone T₃ présente une demi-vie d'au moins 5 jours à une température d'à peu près -30 à 70 °C.

3. Composition conforme à la revendication 1, dans laquelle l'hormone T₃ présente une demi-vie
a) d'au moins un mois ;
b) d'au moins trois mois ;
c) d'au moins six mois ;
d) et/ou d'au moins douze mois.

4. Composition conforme à l'une des revendications précédentes, dans laquelle le rapport de l'hormone T₃ à la sérumalbumine vaut :
a) à peu près de 0,001 à 0,1 ;
b) et/ou de 0,002 à 0,05.

5. Composition conforme à l'une des revendications précédentes, dans laquelle la concentration de l'hormone T₃ vaut :
a) de 0,02 à 0,08 mg/ml ;
b) de 0,1 à 0,5 mg/ml ;
c) ou 0,1 mg/ml.

6. Composition conforme à l'une des revendications précédentes, dont le pH vaut :
a) de 2,5 à 11,5 ;
b) de 4,0 à 10 ;
c) de 6,0 à 8,0 ;
d) ou de 6,5 à 7,5.

7. Composition conforme à l'une des revendications précédentes, qui contient en outre un excipient pharmacologiquement admissible.

8. Composition conforme à la revendication 1, propre à être utilisée
a) en administration par voie intraveineuse ;
b) en administration directe au niveau du coeur ;
c) en administration par voie parentérale ;
d) ou en administration à travers une muqueuse.

9. Composition conforme à la revendication 8, pour laquelle l'administration à travers une muqueuse est choisie parmi les modes d'administration par voie intranasale, par inhalation et par voie orale.

10. Composition conforme à la revendication 9, dans laquelle l'hormone T₃ présente une demi-vie d'au moins deux semaines.

11. Composition conforme à l'une des revendications précédentes, dans laquelle la sérumalbumine est de la sérumalbumine humaine ou de la sérumalbumine bovine.

12. Emploi d'une composition conforme à l'une des revendications précédentes en vue de la fabrication d'un médicament servant à traiter un patient souffrant d'un arrêt cardiaque ou d'une asystolie, afin de rétablir le fonctionnement efficace du coeur.

13. Emploi conforme à la revendication 12, dans lequel
a) l'arrêt cardiaque est provoqué par une dissociation électromécanique ;
b) ou l'asystolie est provoquée par une maladie.

14. Emploi conforme à la revendication 12 ou 13, dans lequel la composition est administrée :
a) par injection directe dans une cavité du coeur du patient ;
b) par injection parentérale directe dans une veine centrale du patient ;
c) par injection parentérale ou injection intraveineuse ;
d) directement dans le système pulmonaire du patient ;
e) directement dans le système pulmonaire par injection endotrachéale directe ;
f) directement dans le système pulmonaire par tubage par les voies respiratoires du patient ;
g) par goutte-à-goutte intraveineux ;
h) ou à travers une muqueuse.

15. Emploi conforme à l'une des revendications 12 à 14, dans lequel la composition est administrée en au moins une injection rapide d'embol.

16. Emploi conforme à l'une des revendications 12 à 15, dans lequel la composition est administrée à une dose
a) de 0,1 à 20 µg d'hormone T₃ par kilogramme de poids corporel ;
b) de 0,2 à 10 µg d'hormone T₃ par kilogramme de poids corporel ;
c) de 0,3 à 5 µg d'hormone T₃ par kilogramme de poids corporel ;
d) et/ou de 100 µg d'hormone T₃ par kilogramme de poids corporel.
